(19) Europäisches Patentamt
European Patent Office
Office européen des brevets

(11) EP 3 546 065 A1

(12) # EUROPÄISCHE PATENTANMELDUNG

(43) Veröffentlichungstag:
**02.10.2019 Patentblatt 2019/40**

(21) Anmeldenummer: **19162229.9**

(22) Anmeldetag: **12.03.2019**

(51) Int Cl.:
***B01J 21/12*** (2006.01) ***B01J 23/04*** (2006.01)
***B01J 23/755*** (2006.01) ***B01J 35/00*** (2006.01)
***B01J 37/00*** (2006.01) ***B01J 37/02*** (2006.01)
***C07C 2/10*** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **14.03.2018 EP 18161757**

(71) Anmelder: **Evonik Degussa GmbH**
**45128 Essen (DE)**

(72) Erfinder:
- **Nadolny, Fabian**
  **59821 Arnsberg (DE)**
- **Peitz, Stephan**
  **45739 Oer-Erkenschwick (DE)**
- **Stochniol, Guido**
  **45721 Haltern am See (DE)**
- **Franke, Robert**
  **45772 Marl (DE)**
- **Quandt, Thomas**
  **45772 Marl (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(54) **OLIGOMERISIERUNGSKATALYSATOR AUF BASIS VON NICKELOXID UND VERFAHREN ZUR OLIGOMERISIERUNG VON OLEFINEN**

(57) Gegenstand der Erfindung ist ein Oligomerisierungskatalysator, welcher Nickeloxid und Silica-Alumina-Supportmaterial enthält, und ein Verfahren zur Oligomerisierung von C3- bis C6-Olefinen unter Verwendung des Oligomerisierungskatalysators.

Der Oligomerisierungskatalysator umfasst Nickeloxid, einen Al-freien und Si-haltigen Binder (< 0,1 Gew.-% Al) und ein röntgenamorphes Silica-Alumina-Supportmaterial und weist eine Zusammensetzung von 15 bis 40 Gew.-% NiO, 5 bis 20 Gew.-% Al2O3 , 55 bis 80 Gew.-% SiO2 und 0,01 bis 1 Gew.-% eines Alkalimetalloxids auf, sowie ein Verhältnis von tetraedrisch koordinierten Gerüst-Aluminiumatomen zu oktaedrisch koordinierten Gerüst-Aluminiumatomen von 75 : 25 bis 100 : 0, bestimmt mittels <27> Al-MAS-NMR.

EP 3 546 065 A1

## Beschreibung

Die vorliegende Erfindung betrifft einen Oligomerisierungskatalysator, welcher Nickeloxid und ein Silica-Alumina-Supportmaterial enthält, und ein Verfahren zur Herstellung des Oligomerisierungskatalysators. Ein weiterer Gegenstand der vorliegenden Erfindung ist ein Verfahren zur Oligomerisierung von C3- bis C6-Olefinen unter Verwendung des Oligomerisierungskatalysators.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Bei den heterogen katalysierten Verfahren ist die Oligomerisierung an sauren Oligomerisierungskatalysatoren lange bekannt. Technisch werden z. B. Zeolithe oder Phosphorsäure auf einem Support eingesetzt. Hierbei werden Isomerengemische von verzweigten Olefinen erhalten. Für die nicht-saure, heterogen katalysierte Oligomerisierung von Olefinen, mit hoher Dimerenselektivität werden in der Technik häufig Nickelverbindungen auf Supportmaterialien eingesetzt. So wird in der WO 95/14647 A1 ein Nickelkatalysator mit einem Supportmaterial, das aus den Komponenten Titanoxid und/oder Zirkoniumoxid, Siliciumdioxid und gegebenenfalls Aluminiumoxid besteht, für die Olefinoligomerisierung beschrieben. An diesen Katalysatoren werden Gemische linearer Butene in einer Selektivität von unter 75 % zu C8-Olefinen oligomerisiert.

WO 95/14647 A1 beschreibt ein Verfahren zur Oligomerisierung von Olefinen mittels eines Oligomerisierungskatalysators, der als aktive Bestandteile, nach Abzug des Glühverlustes nach Temperung bei 900°C, 10 bis 70 Gew.-% Nickeloxid, berechnet als NiO, 5 bis 30 Gew.-% Titandioxid und/oder Zirkoniumoxid, 0 bis 20 Gew.-% Aluminiumoxid, 20 bis 40 Gew.-% Siliciumdioxid und 0,01 bis 1 Gew.-% eines Alkalimetalloxids enthält

Es wird vermutet, dass die katalytische Aktivität von nickelbasierten, heterogenen Katalysatoren zur Oligomerisierung von Olefinen, insbesondere Olefinen mit 3 bis 6 Kohlenstoffatomen, auf der Wechselwirkung zwischen Nickel-Kationen und Oberflächen-Aluminiumatomen basiert. Die Zugabe von Titandioxid und/ Zirkoniumdioxid führt jedoch dazu, dass die Gesamtzusammensetzung prozentual weniger Aluminium bzw. Aluminiumoxid aufweist, was dazu führen kann, dass die katalytische Aktivität und/oder der Umsatz verringert werden. Gleichzeitig kann die Zugabe von Titandioxid und/oder Zirkoniumoxid dazu führen, dass größere Mengen an unerwünschten Oligomerisierungsprodukten, insbesondere stark verzweigten Oligomeren, gebildet werden.

Der vorliegenden Erfindung lag deshalb die Aufgabe zugrunde einen verbesserten Oligomerisierungskatalysator bereitzustellen, welcher die vorgenannten Nachteile nicht aufweist. Ebenso war es die Aufgabe, einen Oligomerisierungskatalysator bereitzustellen, mit dem sich bei der Oligomerisierung höhere Selektivitäten und höhere Umsätze erzielen lassen, wobei kein negativer Effekt auf die Standzeit des Katalysators und die mechanischen Eigenschaften wie Festigkeit erfolgen darf.

Die zugrundeliegende Aufgabe der vorliegenden Erfindung konnte mit dem Oligomerisierungskatalysator gemäß Anspruch 1 und dem Verfahren zur Oligomerisierung nach Anspruch 8 gelöst werden. Bevorzugte Ausführungsformen sind in den Unteransprüchen angegeben.

Der erfindungsgemäße Oligomerisierungskatalysator umfasst Nickeloxid, einen Al-freien und Si-haltigen Binder (Al-frei bedeutet: <0,1 Gew.-% Al in der Gesamtzusammensetzung des Binders), vorzugsweise Siliciumdioxid, und ein amorphes Silica-Alumina-Supportmaterial, vorzugsweise ein amorphes Alumosilicat. Der Binder ist ein Material, das dafür sorgt, dass der erfindungsgemäß hergestellte Katalysator die nötige mechanische Festigkeit aufweist. Mit "röntgenamorph" im Sinne der vorliegenden Erfindung ist die Eigenschaft eines Feststoffes gemeint, die daraus folgt, dass der Feststoff keine Kristallstruktur, d. h. keine Fernordnung, aufweist. Im Sinne der vorliegenden Erfindung ist aber nicht auszuschließen, dass das amorphe Silica-Alumina-Supportmaterial kleine kristalline Domänen aufweist. Das amorphe Silica-Alumina-Supportmaterial ist somit kein kristallines Material, beispielsweise kein zeolithisches Material.

Der Oligomerisierungskatalysator weist dabei eine Zusammensetzung von 15 bis 40 Gew.-%, vorzugsweise 15 bis 30 Gew.-% NiO, 5 bis 20 Gew.-% $Al_2O_3$, 55 bis 80 Gew.-% $SiO_2$ und 0,01 bis 1 Gew.-%, vorzugsweise 0,05 bis 0,5 Gew.-% eines Alkalimetalloxids, vorzugsweise Natriumoxid, auf. Die Angaben beziehen sich auf eine Gesamtzusammensetzung von 100 Gew.-%. Der erfindungsgemäße Oligomerisierungskatalysators weist darüber hinaus ein Verhältnis von tetraedrischen koordinierten Gerüst-Aluminiumatomen zu oktaedrischen koordinierten Gerüst-Aluminiumatomen im Bereich von 75 : 25 bis 100 : 0, vorzugsweise im Bereich von 90 : 10 bis 100 : 0, besonders bevorzugt von 100 : 0, bestimmt mittels $^{27}$Al-MAS-NMR, auf. Die jeweiligen Werte für die oktaedrische und die tetraedrische Koordination im vorgenannten Verhältnis sind als prozentuale Angabe bezogen auf die Gesamtzahl der vorliegenden Aluminiumatome

zu verstehen. In einer besonders bevorzugten Ausführungsform der vorliegenden Erfindung ist der Oligomerisierungskatalysator im Wesentlichen frei von Titandioxid und/oder Zirkoniumoxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und/oder Zirkoniumoxid in seiner Gesamtzusammensetzung.

Die Zusammensetzung und koordinativen Eigenschaften des Oligomerisierungsatalysators betreffen insbesondere den Zustand vor dem Einsatz in der Oligomerisierung, also die Form, in der der Katalysator in die Reaktionszone eingefüllt wird. Der Zustand des Katalysators, insbesondere der Oberflächenatome des Katalysators, lässt sich während der Oligomerisierung nicht abschließend ermitteln. Im vorliegenden Fall zeigt der Katalysator aber auch nach dem Ausbau aus der Reaktionszone eine nahezu identische Zusammensetzung und nahezu identische koordinative Eigenschaften.

Das hier beschriebene Verhältnis der unterschiedlichen Koordinationen der Aluminiumatome betrifft alle Aluminiumatome im Silica-Alumina-Supportmaterial, also sowohl Bulk- als auch Oberflächentatome. Die Bulkstruktur von Alumosilicaten besteht üblicherweise aus $SiO_4$-Tetraedern, $AlO_4$-Tetraedern und optional auch in sehr geringem Umfang $AlO_6$-0ktaedern. An der Oberfläche dieser Struktur fehlt jedoch zur Umgebung hin der Sauerstoff von einem angrenzenden Tetra- oder Oktaeder. Das Siliciumatom an der Oberfläche bildet relativ einfach eine Silanolgruppe aus. Das Aluminiumatom weist jedoch formal nur eine trivalente Koordination mit einem freien Orbital auf. Inklusive des freien Orbitals entspricht dies einer tetraedrischen Koordination. An das freie Orbital können sich Moleküle oder Atome koordinativ binden, beispielsweise Ammoniak (Ammonium-Form, Struktur I der nachfolgenden Abbildung) oder der Sauerstoff einer benachbarten Silanolgruppe (H-Form, Struktur II der nachfolgenden Abbildung). Durch die Wechselwirkung zwischen Aluminium und dem benachbarten Sauerstoff wird die OH-Bindung der benachbarten Silanolgruppe geschwächt und die OH-Gruppe kann als Protonendonor fungieren. Mit Wasser aus der Umgebungsluft wird sich der Sauerstoff aus den Wassermolekülen als Elektronendonor koordinativ an das Aluminiumatom binden. Dabei können sich drei Wassermoleküle anlagern, wodurch eine oktaedrische Koordination entsteht (siehe Struktur III der nachfolgenden Abbildung).

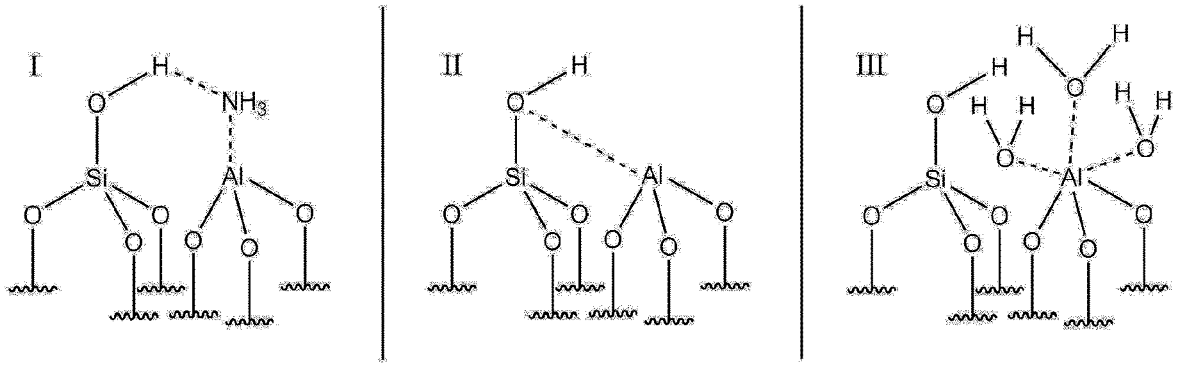

Die koordinativen Eigenschaften der Aluminiumatome des Oligomerisierungskatalysator haben einen Einfluss auf die Oligomerisierung. Im Stand der Technik wird dabei vor allem auf eine überwiegend oktaedrische Koordination der Aluminiumatome abgestellt, so wie es in der US 7,572,946 B2 oder der US 6,733657 B2 beschrieben ist. Im vorliegenden Fall hat sich aber überraschend gezeigt, dass durch die Einstellung des Verhältnisses von tetraedrisch-koordinierten und oktaedrisch-koordinierten Gerüst-Aluminiumatomen auf Werte im Bereich von 75 : 25 bis 100 : 0, vorzugsweise im Bereich von 90 : 10 bis 100 : 0, besonders bevorzugt von 100 : 0besonders gute Katalysatoreigenschaften, wie ein höhere Umsatz und/oder eine höhere Selektivität, beim Einsatz des erfindungsgemäßen Oligomerisierungskatalysators erzielen lassen.

Das Verhältnis von tetraedrisch koordinierten Aluminiumatomen zu oktaedrisch koordinierten Aluminiumatomen kann mittels $^{27}$Al-MAS-NMR bestimmt werden, wobei die tetraedrische Koordination der Aluminiumatome bei 50 bis 60 ppm und die oktaedrische Koordination der Aluminiumatome bei 0 ppm detektiert werden und mittels Integration des Peaks ausgewertet werden können. Die genaue Messung der NMR-Spektren wurde wie folgt durchgeführt: Dazu erfolgt zunächst die Probenvorbereitung, bei der die zu untersuchenden Proben vor den Messungen etwa 2 Stunden in wasserdampfgesättigter Atmosphäre gelagert (Exsikkator, Proben bei Raumtemperatur 2 bis 3 cm über destilliertem Wasser). Dadurch wird die Signalintensität erhöht und sichergestellt, dass alle Proben den gleichen Ausgangspunkt haben. Danach werden je nach Dichte 300 bis 500 mg Probenmaterial in einen $ZrO_2$-Festkörper-Spinner (Außendurchmesser 4 mm) gegeben und mit leichtem Druck verdichtet.

Die eigentlichen NMR-Messungen werden an einem Bruker 400 Avance III HD Spektrometer mit einem speziellen Festkörper-Probenkopf durchgeführt. Zur Eliminierung der Anisotropie werden bei Festkörper-Messungen die Proben um ca. 54° geneigt und in schnelle Rotation versetzt (bei $^{27}$Al z.B. 12 kHz). Zur Signalanregung wird ein einfacher 90° Puls verwendet. Aufgrund der sehr schnellen Relaxation von $^{27}$Al Kernen wird mit einem sehr kurzen Delay von ca.

50 ms gemessen, so dass eine sehr schnelle Pulsfolge möglich ist. Die Anregungsbreite (sweep Weite) beträgt 400 ppm, wobei der Sender auf 0 ppm gesetzt wird (Messbereich + 200 bis -200 ppm).

Die Auswertung der Spektren wird wie folgt durchgeführt. Festkörper Spektren von Quadrupolkernen ($^{27}$Al ist ein Quadrupolkern (Kernspin I=5/2)) weisen oft schwer korrigierbare Basislinien auf. Hierfür ist, nach erfolgter Phasenkorrektur, eine Basislinienkorrektur mit einem Polynom in den meisten Fällen geeignet. Die in äquidistantem Abstand auftretenden Rotationsseitenseitenbanden (Abstand 12 kHz) liegen außerhalb des Auswertebereichs und haben somit keinen Einfluss auf die Auswertung. Die relevanten Signale werden unter Berücksichtigung der jeweiligen Linienbreiten integriert und können zur Bestimmung der prozentualen Anteile (%Al) zueinander ins Verhältnis gesetzt werden. Die Spektren werden auf eine gesättigte $Al(NO_3)_3$-Lösung referenziert ($\delta$=0,0 ppm). Eine tetraedrische Koordination der Aluminiumatome wird im NMR-Spektrum Bereich von 50 bis 60 ppm detektiert. Eine oktaedrische Koordination der Aluminiumatome erzeugt im NMR-Spektrum einen Peak um 0 ppm.

Erfindungsgemäß kann der Oligomerisierungskatalysator zudem eine spezifische Oberfläche (berechnet nach BET) von 150 bis 400 $m^2$/g, vorzugsweise 190 bis 350 $m^2$/g, besonders bevorzugt von 220 bis 330 $m^2$/g auf. Die spezifische Oberfläche wird mittels Stickstoff-Physisorption gemäß DIN-ISO 9277 (Stand: 2014-01) gemessen bzw. daraus errechnet.

In einer weiterhin bevorzugten Ausführungsform weist der Oligomerisierungskatalysator Mesoporen und Makroporen auf, hat also eine bimodale Porengrößenverteilung. Die Mesoporen des erfindungsgemäßen Oligomerisierungskatalysators weisen einen mittleren Porendurchmesser von 5 bis 15 nm, vorzugsweise von 7 bis 14 nm, besonders bevorzugt von 9 bis 13 nm auf. Die Makroporen des erfindungsgemäßen Oligomerisierungskatalysators weisen demgegenüber vorzugsweise einen mittleren Porendurchmesser von 1 bis 100 $\mu$m, besonders bevorzugt von 2 bis 50 $\mu$m auf. Das mittlere Porenvolumen des erfindungsgemäßen Oligomerisierungskatalysators, d. h. sowohl der Mesoporen als auch der Makroporen, kann 0,5 bis 1,5 $cm^3$/g, vorzugsweise 0,7 bis 1,3 $cm^3$/g betragen. Der mittlere Porendurchmesser und das mittlere Porenvolumen können mittels Quecksilber-Porosimetrie gemäß DIN 66133 (Stand: 1993-06) bestimmt werden.

Der erfindungsgemäße Oligomerisierungskatalysator liegt vorzugsweise als Granulat vor. Darüber hinaus kann der erfindungsgemäße Oligomerisierungskatalysator einen mittleren Partikeldurchmesser (d50) von 0,1 mm bis 7 mm, vorzugsweise 0,5 bis 6 mm, besonders bevorzugt von 1 mm bis 5 mm aufweisen. Der mittlere Partikeldurchmesser kann mittels bildgebender Verfahren ermittelt werden, insbesondere durch die in den Normen ISO 13322-1 (Stand: 2004-12-01) und ISO 13322-2 (Stand: 2006-11-01) genannten Verfahren. Ein geeignetes Gerät zur Analyse des Partikeldurchmessers ist beispielsweise der Camsizer 2006 (Retsch Technology).

Der Oligomerisierungskatalysator weist in einer weiteren bevorzugten Ausführungsform eine Bulk Crush Strength (BCS) von mehr als 0,5 MPa, vorzugsweise von mehr als 0,6 MPa und besonders bevorzugt von mehr als 0,8 MPa auf. Der BCS-Wert ist ein Maß für die mechanische Festigkeit mineralischer Granulate. Die Bulk Crush Strength (BCS) eines Feststoffes ist ein als Druck in MPa definierter Parameter zu verstehen, bei welchem 0,5 Gew.-% Feinanteil (d.h. Teilchen, die über ein Sieb mit einer Maschenweite von 0,425 mm abgesiebt wurden) gebildet werden, wenn die Feststoffprobe über einen Kolben in einem Rohr mit Druck beaufschlagt wird. Für diesen Zweck werden 20 ml des Feststoffes mit einem Sieb (Maschenweite: 0,425 mm) vorgesiebt, in ein zylindrisches Probenrohr (Innendurchmesser: 27,6 mm, Wandstärke: 5 mm, Höhe: 50 mm) eingefüllt und 5 ml Stahlkugeln (Durchmesser: 3,9 mm) auf die Oberseite des Feststoffes gegeben. Der Feststoff wird anschließend mit unterschiedlichen (zunehmenden) Drücken für drei Minuten beaufschlagt. Anschließend werden die durch die Beaufschlagung mit Druck entstandenen Feinanteile per Siebung abgetrennt, jeweils in Summe gewogen und ihr Prozentanteil bestimmt. Dieses Verfahren wird durchgeführt, bis eine Menge von 0,5 Gew.-% Feinanteil erreicht wird.

Ein Oligomerisierungskatalysator kann auch über seine maximale Schüttdichte charakterisiert werden. In einer bevorzugten Ausführungsform weist der erfindungsgemäße Oligomerisierungskatalysator eine maximale Schüttdichte von 0,1 bis 2 g/$cm^3$, vorzugsweise 0,2 bis 1,5 g/$cm^3$, besonders bevorzugt von 0,3 bis 1,0 g/$cm^3$ auf. Die Bestimmung der Schüttdichte kann über einen Messzylinder erfolgen. In den Messzylinder wird ein bestimmtes Volumen des zu untersuchenden Feststoffes eingefüllt, beispielsweise über eine geeignete Dosiervorrichtung wie den DR100 (Retsch), und der Messzylinder gewogen. Aus dem Gewicht und dem Volumen lässt sich die maximale Schüttdichte ermitteln. Gegebenenfalls muss die Restfeuchte von dem Probengewicht abgezogen werden.

Der erfindungsgemäße Oligomerisierungskatalysator wird durch ein Verfahren hergestellt, welches die folgenden Schritte umfasst:

a) Mischen des amorphen Silica-Alumina-Supportmaterials und des Al-freien und Si-haltigen Binders optional zusätzlich mit mindestens einem Teil einer Nickelquelle, und Granulieren der so hergestellten Mischung;

b) Beaufschlagen (Imprägnieren) des in Schritt a) hergestellten Granulats mit mindestens einem Teil einer Nickelquelle, sofern die gesamte Nickelquelle nicht schon in Schritt a) mit dem Silica-Alumina-Supportmaterial und dem amorphen Al-freien und Si-haltigen Binder vermischt worden ist; und

c) Kalzinieren des Granulats zur Herstellung des Oligomerisierungskatalysators.

Das in Schritt a) eingesetzte amorphe Silica-Alumina-Supportmaterial weist im kalzinierten Zustand (ohne Ni) ein Verhältnis von tetraedrischen koordinierten Gerüst-Aluminiumatomen zu oktaedrischen koordinierten Gerüst-Aluminiumatomen von 50 : 50 bis 74 : 26, vorzugsweise 55 : 45 bis 70 : 30 auf. Das Verhältnis der Koordination von tetraedrischen koordinierten Gerüst-Aluminiumatomen zu oktaedrischen koordinierten Gerüst-Aluminiumatomen im Silica-Alumina-Supportmaterial weicht also von dem gleichen Verhältnis beim finalen Oligomerisierungskatalysator ab und ändert sich also während der Herstellung des Katalysators. Dabei wird der Anteil der tetraedrischen Koordination in dem erwähnten Verhältnis ausgehend vom eingesetzten Silica-Alumina-Supportmaterial hin zum finalen Oligomerisierungskatalysator ansteigen, während der Anteil der oktaedrischen Koordination sinkt.

Das amorphe Silica-Alumina-Supportmaterial ist ein amorphes Alumosilicat, welches 10 bis 20 Gew.-%, vorzugsweise 12 bis 17 Gew.-% $Al_2O_3$ und 80 bis 90 Gew.-%, vorzugsweise 83 bis 88 Gew.-% $SiO_2$ umfasst. Die Angabe bezieht sich auf die Zusammensetzung ohne eventuell sorbierte Verbindungen (z. B. Wasser oder Ammoniak), die beispielsweise unter dem Begriff Glühverlust in kommerziell erhältlichen Produkten angegeben werden. In einer bevorzugten Ausführungsform kann das als das Silica-Alumina-Supportmaterial eingesetzte amorphe Alumosilicat eine Korngröße (d50) im Bereich von 10 bis 80 $\mu$m, vorzugsweise 15 bis 75 $\mu$m aufweisen. Darüber hinaus weist das als das Silica-Alumina-Supportmaterial eingesetzte amorphe Alumosilicat vorzugsweise eine spezifische Oberfläche (berechnet nach BET) von 290 bis 380 $m^2/g$, besonders bevorzugt von 300 bis 360 $m^2/g$ auf, gemessen mittels Stickstoff-Physisorption gemäß DIN-ISO 9277 (Stand: 2014-01). Der Anteil des Silica-Alumina-Supportmaterials an dem Gesamtansatz (Gesamtzusammensetzung inklusive aller ggf. verwendeten Lösemittel wie Wasser) in Schritt a) beträgt 20 bis 60 Gew.-%, vorzugsweise 25 bis 50 Gew.-%, wenn die gesamte Nickelquelle bereits in Schritt a) zugegeben wird. Wird die Nickelquelle teilweise oder vollständig erst in Schritt b) zugegeben, sollte eine ausreichende Flüssigkeitsmenge durch Zugabe eines Lösemittels, vorzugsweise Wasser oder eine ammoniakalische Lösung, zur Mischung in Schritt a) hinzugefügt werden, um eine Granulation zu ermöglichen.

Der in Schritt a) ebenfalls eingesetzte Al-freie und Si-haltige Binder (Al-frei bedeutet: <0,1 Gew.-% Al in der Gesamtzusammensetzung des Binders) ist vorzugsweise Siliciumdioxid. Der Al-freie und Si-haltige Binder liegt weiterhin bevorzugt nicht in fester Form, sondern in Form einer kolloidalen Dispersion, besonders bevorzugt einem Kieselsol, vor. Das Lösemittel, in dem der Al-freie und Si-haltige Binder, vorzugsweise das Siliciumdioxid, dispergiert vorliegt, ist in einer bevorzugten Ausführungsform Wasser, vorzugsweise enthält die Dispersion zusätzlich Ammoniak zur Stabilisierung. Der Al-freie und Si-haltige Binder, vorzugsweise das Siliciumdioxid, ist in der Dispersion in einer Menge im Bereich von 7 bis 50 Gew.-%, vorzugsweise 12 bis 42 Gew.-%, besonders bevorzugt 20 bis 35 Gew.-% vorhanden. Die mittlere Partikelgröße des Al-freien und Si-haltigen Binders, vorzugsweise des Siliciumdioxids, kann, insbesondere in der Dispersion, 5 bis 20, vorzugsweise 6 bis 10 nm betragen (kann mittels Lichtstreumethoden ermittelt werden). Die Viskosität der Dispersion mit dem Al-freien und Si-haltigen Binder, vorzugsweise dem Siliciumdioxid, kann im Bereich von 1 bis 50 mPas, vorzugsweise 5 bis 25 mPas liegen. Weiterhin bevorzugt kann die Dispersion mit dem Al-freien und Si-haltigen Binder, vorzugsweise dem Siliciumdioxid einen pH-Wert im Bereich von 7 bis 12, vorzugsweise 8 bis 10 aufweisen. Die Dichte der Dispersion mit dem Al-freien und Si-haltigen Binder, vorzugsweise dem Siliciumdioxid, beträgt vorzugsweise 1 bis 1,3 $g/cm^3$, besonders bevorzugt 1,1 bis 1,25 $g/cm^3$. Der Anteil des Al-freien und Si-haltigen Binders an dem Gesamtansatz (Gesamtzusammensetzung inklusive aller ggf. verwendeten Lösemittel wie Wasser) in Schritt a) und optional b) beträgt 0,5 bis 15 Gew.-%, vorzugsweise 1 bis 10 Gew.-%.

Zur Mischung in Schritt a) oder b) wird keine separate Alkaliquelle hinzugefügt. Der Gehalt an Alkalimetalloxid entstammt dem Silica-Alumina-Trägermaterial und/oder dem Al-freien und Si-haltigen Binder, die geringe Mengen an Alkalimetallen, insbesondere Natrium, enthalten können. Die Alkaligehalte von Trägermaterial und Binder sind so zu wählen, dass ein Anteil von 0,01 Gew.-% nicht unterschritten und ein Anteil von 1 Gew.-% nicht überschritten wird.

Als Nickelquelle in Schritt a) oder b) wird eine Lösung einer Nickelverbindung, eine Paste aus einer Nickelverbindung oder eine Lösung und eine Paste eingesetzt, wobei prinzipiell jede lösliche Nickelverbindung eingesetzt werden kann. Darunter fallen Nickelnitrat ($Ni(NO_3)_2$), Nickelacetat ($Ni(ac)_2$), Nickelacetylacetonat ($Ni(acac)_2$), Nickelsulfat ($NiSO_4$), Nickelcitrat oder Nickelcarbonat ($NiCO_3$). Bevorzugt sind Nickelnitrat ($Ni(NO_3)_2$), Nickelsulfat ($NiSO_4$) oder Nickelcarbonat ($NiCO_3$). Die Nickel-Lösung ist eine wässrige oder ammoniakalische Lösung. Eine ammoniakalische Lösung ist eine wässrige Lösung, welche mit Ammoniak versetzt wurde. Die Nickel-Paste enthält Wasser, wobei die Nickel-Paste gemäß der vorliegenden Erfindung weniger Wasser enthält als die Nickel-Lösung (wenn man von der gleichen Menge an Nickelverbindung ausgeht). Nickel-Paste ist prinzipiell ein angefeuchteter Feststoff aus einer Nickelverbindung, die unvollständig hydratisiert ist und bei der sich formal auch hydroxidische Nickelverbindung bilden, bei Nickelcarbonat beispielsweise $NiCO_3*Ni(OH)_2$, aber auch nicht-stöchiometrische Nickelcarbonat-Hydroxide. Die Nickel-Paste enthält in einer bevorzugten Ausführungsform zwischen 30 und 50 Gew.-%, vorzugsweise 35 bis 45 Gew.-% Nickel bezogen auf das Gesamtgewicht der Paste. Die Nickellösung kann Nickel in einer Menge im Bereich von 1 bis 20 Gew.-%, vorzugsweise 5 bis 15 Gew.-%, enthalten, jeweils bezogen auf das Gesamtgewicht der Lösung.

In einer bevorzugten Ausführungsform wird als Nickel-Lösung eine ammoniakalische $Ni(CO_3)$-Lösung, genannt

NiHAC-Lösung (es bildet sich in der Lösung ein Nickelhexamincarbonat-Komplex ($[Ni(NH_3)_6]CO_3$)), verwendet, die einen Nickelgehalt im Bereich von 1 bis 20 Gew.-%, vorzugsweise 5 bis 15 Gew.-% aufweist. Als Nickel-Paste kann eine Paste aus Nickelcarbonat und Wasser als Lösemittel verwendet werden, wobei das Nickel als Carbonat/Hydroxid vorliegt (allgemeine Summenformel $NiCO_3{*}Ni(OH)_2$, es können aber auch nicht-stöchiometrische Nickelcarbonat-Hydroxide gebildet werden). Die Paste kann einen Nickelgehalt im Bereich von 30 und 50 Gew.-%, vorzugsweise 35 bis 45 Gew.-% aufweisen. In einer besonders bevorzugten Ausführungsform wird bei der Herstellung des Oligomerisierungskatalysators in Schritt a) und/oder im optionalen Schritt b) eine NiHAC-Lösung verwendet.

Die Gesamtmenge an Nickelquelle (Paste und/oder Lösung) an dem Gesamtansatz (Gesamtzusammensetzung aller ggf. verwendeten Lösemittel wie Wasser) in Schritt a) und optional b) des Herstellverfahrens beträgt zwischen 40 und 70 Gew.-%, vorzugsweise 45 und 65 Gew.-%.

Das erfindungsgemäße Verfahren zeichnet sich insbesondere dadurch aus, dass in Schritt a) kein Titandioxid und kein Zirkoniumdioxid zur Mischung gegeben wird, sondern der Oligomerisierungskatalysator ohne Hinzufügen von Titandioxid und Zirkoniumoxid hergestellt wird. Mögliche Vorkommen von Titandioxid und/oder Zirkoniumdioxid in der Gesamtzusammensetzung des Oligomerisierungskatalysators stammen aus Verunreinigungen bzw. Spurenvorkommen in den eingesetzten Komponenten.

Im Schritt a) werden die einzelnen Komponenten, d. h. das Silica-Alumina-Supportmaterial, der Al-freie und Si-haltige Binder und optional die Nickelquelle, miteinander in einem Mischbehälter unter Verwendung eines Wirblers vermischt und gleichzeitig oder anschließend granuliert. Dazu kann beispielsweise ein Intensivmischer verwendet werden. Die Vermischung und die Granulation können typischerweise bei Umgebungsdruck durchgeführt werden. Die Temperatur, bei der Vermischung und Granulation stattfinden können, liegt vorzugsweise im Bereich von 10 bis 60 °C. Die Dauer des Verfahrensschritts a), also der Vermischung und der Granulation, beträgt zwischen 5 Minuten und 1 Stunde, vorzugsweise zwischen 10 und 30 Minuten.

Im optionalen Schritt b) wird der restliche Teil der Nickelquelle, vorzugsweise in Form einer NiHAC-Lösung, zu dem in Schritt a) hergestellten Granulat gegeben und mit dem Granulat vermischt, um das Granulat mit Nickel zu beaufschlagen. Sollte zumindest ein Teil der Nickelquelle in Schritt b) zugegeben werden, kann das ggf. feuchte Granulat aus Schritt a) vor dem Beaufschlagen mit der Nickelquelle getrocknet werden. Die Trocknungstemperatur kann 80 bis 250 °C, vorzugsweise 100 bis 220 °C betragen.

Durch die Nickelquelle wird auf der Oberfläche des Silica-Alumina-Trägermaterials eine Nickelverbindung abgeschieden. Dadurch werden vorher oktaedrische koordinierte Aluminiumatome an der Oberfläche zumindest teilweise in tetraedrischer Koordination fixiert und können nach der Kalzination keine oktaedrische Koordination mehr einnehmen. Der Binder wird als die unterschiedlichen Materialien verbindender Stoff hinzugefügt und ist hier Al-frei, um sicherzustellen, dass keine zusätzlichen oktaedrisch koordinierten Aluminiumatome in den Katalysator eingebracht werden. Gegebenenfalls vorhandene oktaedrisch koordinierte Aluminiumatome im Bulk des Trägermaterials bleiben davon unberührt, weshalb auch nach Vermischung mit dem Binder unter Umständen eine gewisse Menge an oktaedrisch koordinierten Aluminiumatomen im fertigen Oligomerisierungskatalysator vorhanden ist.

Das aus Schritt a) und/oder Schritt b) resultierende Granulat kann noch mindestens einen Teil der verwendeten Lösemittel, insbesondere Wasser, enthalten. Es kann sich also um ein feuchtes Granulat handeln. Bevor das gegebenenfalls noch feuchte Granulat der Kalzination in Schritt c) unterworfen wird, kann das feuchte Granulat gesiebt werden, vorzugsweise mit einem Sieb mit einer Maschenweite von 0,1 bis 1,5 mm. Der abgesiebte Teil des Granulats (Unterkorn) kann zurück zu Schritt a) der Granulation zurückgeführt werden.

Nach dem Vermischen und Granulieren in Schritt a), optional nach dem Beaufschlagen (Imprägnieren) des Granulats mit mindestens einem Teil einer Nickelquelle in Schritt b) und optional nach dem Sieben des feuchten Granulats kann das Granulat in Schritt c) zunächst getrocknet werden. Dazu können bekannte Geräte wie beispielsweise Bandtrockner oder ähnliches verwendet werden. Die Trocknungstemperatur kann im Bereich von 80 bis 250 °C, vorzugsweise im Bereich von 100 bis 220 °C liegen.

Bevor das optional getrocknete Granulat der Kalzination unterworfen wird, kann das getrocknete Granulat fraktioniert werden, um eine bestimmte Korngröße des Granulats einzustellen. Eine solche Fraktionierung kann beispielsweise durch die Verwendung mindestens eines Siebs mit einer definierten Maschenweite erreicht werden. In einer besonders bevorzugten Ausführungsform werden zwei Siebe verwendet, wobei das eine Sieb eine Maschenweite von 0,1 bis 1,5 mm und das andere Sieb eine Maschenweite von 2,5 bis 7 mm aufweist. Die übrigen Fraktionen (Ober- und Unterkorn) können ggf. nach vorheriger Mahlung zum Schritt a) zurückgeführt werden.

Nach der optionalen Trocknung und eventuellen Fraktionierung des Granulats erfolgt die Kalzination des Granulats. Dabei kann das Granulat in einem geeigneten Ofen, vorzugsweise im Stickstoffstrom, besonders bevorzugt Stickstoffgegenstrom, erhitzt werden. Dem Stickstoffstrom kann während der Kalzination Luft hinzugefügt werden, wobei die Menge der zugeführten Luft 100 bis 10000 Vol.-ppm (part per million volumetrisch), vorzugsweise 300 bis 7000 Vol.-ppm betragen kann. Die Kalzinationstemperatur kann 400 bis 900 °C, vorzugsweise 450 bis 700 °C, besonders bevorzugt 500 bis 600 °C betragen. Diese Temperatur kann über mehrere Stunden, vorzugsweise 5 bis 20 Stunde, besonders bevorzugt 8 bis 15 Stunden gehalten werden, bevor das Granulat abgekühlt wird. Beim Abkühlen kann in

den Ofen Luft eingeleitet werden, die Menge der eingeleiteten Luft sollte aber kontrolliert werden. Die Menge der optional zugeführten Luft beträgt 100 bis 10000 Vol.-ppm, vorzugsweise 300 bis 7000 Vol.-ppm.

Danach kann das abgekühlte Granulat bzw. der fertige Oligomerisierungskatalysator, möglicherweise noch einmal fraktioniert werden, um eine bestimmte Korngröße des abgekühlten Granulats einzustellen. Eine solche Fraktionierung kann beispielsweise durch die Verwendung mindestens eines Siebs mit einer definierten Maschenweite erreicht werden. In einer besonders bevorzugten Ausführungsform werden zwei Siebe verwendet, wobei das eine Sieb eine Maschenweite von 0,1 bis 1,5 mm und das andere Sieb eine Maschenweite von 2,5 bis 7 mm aufweist. Die übrigen Fraktionen (Ober- und Unterkorn) können ggf. nach vorheriger Mahlung zum Schritt a) zurückgeführt werden.

Der so hergestellte Oligomerisierungskatalysator weist nach dem letzten Verfahrensschritt, der Kalzination bzw. einer nachgeschalteten Fraktionierung nach Abkühlung, eine finale Gesamtzusammensetzung von 15 bis 40 Gew.-%, vorzugsweise 15 bis 30 Gew.-% NiO, 10 bis 30 Gew.-% $Al_2O_3$, 55 bis 70 Gew.-% $SiO_2$ und 0,01 bis 1 Gew.-%, vorzugsweise 0,05 bis 0,5 Gew.-% eines Alkalimetalloxids auf. Die Angaben beziehen sich auf eine Gesamtzusammensetzung von 100 Gew.-%.

Mit steigender Einsatzzeit des Oligomerisierungskatalysators bei der Oligomerisierung kann es zu einer Abnahme des Umsatzes und/oder der Selektivität kommen. Der erfindungsgemäße Katalysator kann nach dem Einsatz in der Oligomerisierungsreaktion regeneriert werden.

Die Regenerierung des Oligomerisierungskatalysators umfasst die Schritte:

d) Abbrand; und

e) Wiederherstellung der aktiven Oberflächenstruktur des Oligomerisierungskatalysators.

Es ist möglich, dass der Oligomerisierungskatalysator nach seinem Einsatz in Oligomerisierungsreaktionen Ablagerungen organischer Stoffe aufweist, die entfernt werden müssen. Die Entfernung der im Katalysator abgelagerten organischen Verbindungen geschieht vorzugsweise in Schritt d) durch Abbrand (Oxidation), wodurch Kohlenstoffoxide und Wasser entstehen. Der Abbrandschritt d) kann in einem Ofen, beispielweise in einem Drehrohrofen oder einem Schachtofen, kontinuierlich oder diskontinuierlich durchgeführt werden. Dazu wird der Oligomerisierungskatalysators (in Form eines Granulats) dem Ofen zugeführt und vorzugsweise bei einer vorgegebenen Ofentemperatur von 400 bis 600 °C, besonders bevorzugt von 500 bis 600 °C gehalten. Die beim Abbrand verwendete Verbrennungsluft wird im Gegenstrom zugeführt, optional wird zusätzlich weitere Luft über geeignete Einlässe in das Granulat (Oligomerisierungskatalysator) eingeblasen, um einen schnellen Abbrand zu gewährleisten.

Schritt e), also die Wiederherstellung der aktiven Oberflächenstruktur des Oligomerisierungskatalysators kann in einem Schritt e1) eine (zusätzliche) Beaufschlagung (Imprägnierung) mit Nickel umfassen. Die Beaufschlagung mit Nickel kann analog zur Herstellung des Oligomerisierungskatalysators (Schritt b)) erfolgen, optional jedoch mit dem Unterschied, dass eine Nickel-Lösung mit einer geringeren Nickelkonzentration als bei der Herstellung des Oligomerisierungskatalysators verwendet werden kann. Eine Nickel-Paste wird üblicherweise nicht für die Regenerierung eingesetzt. Dabei kommt es darauf an, zusätzliche Mengen an Nickel auf dem Oligomerisierungskatalysator abzuscheiden. Prinzipiell kann dazu jede lösliche Nickelverbindung wie Nickelnitrat ($Ni(NO_3)_2$), Nickelacetat ($Ni(ac)_2$), Nickelacetylacetonat ($Ni(acac)_2$), Nickelsulfat ($NiSO_4$) oder Nickelcarbonat ($NiCO_3$) zur Herstellung einer wässrigen oder ammoniakalischen Nickel-Lösung verwendet werden.

Als besonders vorteilhaft hat sich die Verwendung von NiHAC-Lösungen erwiesen, die durch Auflösen von Nickelcarbonat ($NiCO_3$) in konzentrierten Ammoniaklösungen ggf. mit Zusatz von Ammoniumcarbonat erhältlich sind. Derartige Lösungen können mit Nickelgehalten von 0,5 bis 14 Gew.-%, insbesondere von 2 bis 10 Gew.-%, ganz besonders von 4 bis 8 Gew.-% für die Imprägnierung verwendet werden.

Zum Nickelauftrag wird der in Schritt d) abgebrannte Oligomerisierungskatalysator beispielsweise mit einer NiHAC-Lösung mit Nickelgehalten von 0,5 bis 14 Gew.-%, insbesondere von 2 bis 10 Gew.-%, ganz besonders von 4 bis 8 Gew.-% bis zur Sättigung der Poren imprägniert. Die Imprägnierung kann mit einem dem Fachmann geläufigen Verfahren wie beispielsweise durch Besprühen bis zum permanenten Auftreten eines Flüssigkeitsfilmes auf der Oberfläche (incipient wetness) durchgeführt werden. Beträgt die Lösungsaufnahme etwa 0,8 bis 1,2 g Lösung pro g Oligomerisierungskatalysator, kann man erreichen, dass etwa 0,5 bis 6 Gew.-% an zusätzlichem Nickel in Form eines basischen Carbonates abgeschieden werden.

Wird der Oligomerisierungskatalysator einem Schritt e1) unterworfen, d.h. mit Nickel beaufschlagt, sollte der Oligomerisierungskatalysator in einer geeigneten Trocknungsapparatur, beispielsweise einem Bandtrockner mit Luftstrom oder auch einem Konustrockner, bei Temperaturen zwischen 80 und 250 °C, vorzugsweise zwischen 100 und 220 °C und Normaldruck oder auch im Vakuum getrocknet werden.

Schritt e) umfasst mindestens den Schritt e2) die Kalzination, die nach einem optionalen Schritt e1) durchgeführt werden würde. Die Kalzination des Oligomerisierungskatalysators kann in einem geeigneten Ofen, wie beispielsweise einem Schachtofen oder Drehrohrofen, kontinuierlich oder diskontinuierlich durchgeführt werden. Bei einer kontinuierlichen Kalzination in Schritt e2) wird weiterhin bevorzugt ein Gas im Gegenstrom durch den Oligomerisierungskatalysator (Granulat) geleitet. Als Gas kann Luft, Stickstoff oder eine Mischung davon verwendet werden. Der Gasstrom kann 0,2 bis 4 $m^3$ Gas pro kg Granulat und Stunde und die Eintrittstemperatur des Gases von 400 bis 800 °C, vorzugsweise 450 bis 700 °C betragen. Zusätzlich zu dieser über das Gas eingetragenen Wärme kann Energie durch aktives Heizen der Wände des Ofens eingetragen werden.

Die Kalzinationstemperatur in dem Ofen kann 400 bis 800 °C, vorzugsweise 450 bis 700 °C, besonders bevorzugt 500 bis 600 °C betragen. Diese Temperatur kann über mehrere Stunden, vorzugsweise 5 bis 60 Stunden, besonders bevorzugt 10 bis 40 Stunden gehalten werden, bevor das Granulat abgekühlt wird. Das Abkühlen findet vorzugsweise im Stickstoffstrom statt. Dem Stickstoff kann zusätzlich Luft hinzugefügt werden, wobei die Luftmenge vorzugsweise kontrolliert werden sollte. Die Menge an Luft, die dem Stickstoff vorzugsweise hinzugefügt wird, kann 100 bis 10000 Vol.-ppm, bevorzugt 300 bis 7000 Vol.-ppm betragen.

Der erfindungsgemäße Oligomerisierungskatalysator bzw. der mit dem erfindungsgemäßen Verfahren hergestellte oder regenerierte Katalysator kann insbesondere für die Oligomerisierung von C3- bis C6-Olefinen, vorzugsweise C3- bis C5-Olefinen, besonders bevorzugt C4-Olefinen oder darauf basierenden olefinhaltigen Einsatzgemischen verwendet werden. Die Olefine oder olefinhaltigen Einsatzgemische werden als Eduktstrom eingesetzt.

Gegenstand der vorliegenden Erfindung ist auch ein Verfahren zur Oligomerisierung von C3- bis C6-Olefinen, wobei olefinhaltiges Einsatzgemisch, welches die C3- bis C6-Olefine enthält, in mindestens einer Reaktionszone über einen Katalysator geleitet wird, wobei der erfindungsgemäße Oligomerisierungskatalysator zur Katalyse der Oligomerisierungsreaktion eingesetzt wird.. Eine Reaktionszone umfasst erfindungsgemäß mindestens einen Reaktor und mindestens ein Destillationskolonne, in der die gebildeten Oligomere abgetrennt werden können. Das erfindungsgemäße Verfahren kann auch mit zwei oder mehr Reaktionszonen betrieben werden. Die Oligomerisierung findet vorzugsweise in flüssiger Phase statt.

Als Olefine für das erfindungsgemäße Verfahren werden C3- bis C6-Olefine, bevorzugt C3 bis C5-Olefine, besonders bevorzugt C4-Olefine oder darauf basierende olefinhaltige Einsatzgemische, die auch Anteile an analogen Alkanen enthalten können, eingesetzt. Geeignete Olefine sind unter anderem $\alpha$-Olefine, n-Olefine und Cycloalkene. Bevorzugt sind die als Edukte eingesetzten Olefine n-Olefine. In einer besonders bevorzugten Ausführungsform handelt es sich bei dem Olefin um n-Buten. Der Begriff, darauf basierende olefinhaltige Einsatzgemische ist erfindungsgemäß so zu verstehen, dass er jegliche Art von Gemischen betrifft, die die entsprechenden zu oligomerisierenden C3- bis C6-Olefine in einer Menge enthalten, die es ermöglicht, die Oligomerisierung durchzuführen. Bevorzugt enthalten die olefinhaltigen Einsatzgemische praktisch keine weiteren ungesättigten Verbindungen und mehrfach ungesättigte Verbindungen wie Diene oder Acetylenderivate. Vorzugsweise werden olefinhaltige Einsatzgemische eingesetzt, die weniger als 5 Gew.-%, insbesondere weniger als 2 Gew.-% an verzweigten Olefinen bezogen auf den Olefinanteil enthalten. Weiterhin bevorzugt werden olefinhaltige Einsatzgemische eingesetzt, die weniger als 2 Gew.-% verzweigte Olefine, insbesondere iso-Olefine, enthalten.

Propylen (C3) wird großtechnisch durch Spaltung von Naphtha hergestellt und ist eine Grundchemikalie, die leicht verfügbar ist. $C_5$-Olefine sind in Leichtbenzinfraktionen aus Raffinerien oder Crackern enthalten. Technische Gemische, die lineare $C_4$-Olefine enthalten, sind Leichtbenzinfraktionen aus Raffinerien, $C_4$-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. Beispielsweise können für das erfindungsgemäße Verfahren geeignete Gemische linearer Butene aus der $C_4$-Fraktion eines Steamcrackers gewonnen werden. Dabei wird im ersten Schritt Butadien entfernt. Dies geschieht entweder durch Extraktion oder Extraktionsdestillation des Butadiens oder dessen Selektivhydrierung. In beiden Fällen wird ein praktisch butadienfreier $C_4$-Schnitt erhalten, das Raffinat I. Im zweiten Schritt wird Isobuten aus dem $C_4$-Strom entfernt, z. B. durch Herstellung von Methyl-tert.-butylether (MTBE) durch Umsetzung mit Methanol. Andere Möglichkeiten sind die Umsetzung des Isobutens aus dem Raffinat I mit Wasser zu tert.-Butanol oder die sauer katalysierte Oligomerisierung des Isobutens zu Diisobuten. Der jetzt isobutenfreie $C_4$-Schnitt, das Raffinat II, enthält wie gewünscht die linearen Butene und gegebenenfalls Butane. Optional kann noch das 1-Buten destillativ abgetrennt werden. Beide Fraktionen, die mit But-1-en oder die mit But-2-en, können im erfindungsgemäßen Verfahren eingesetzt werden.

In einer weiteren bevorzugten Ausführungsform werden $C_4$-olefinhaltige Stoffströme dem Verfahren als olefinhaltige Einsatzgemische zugeführt. Geeignete olefinhaltige Einsatzgemische sind unter anderem das Raffinat I (butadienfreier C4-Schnitt des Steamcrackers) sowie das Raffinat II (butadien- und isobutenfreier C4-Schnitt des Steamcrackers).

Eine weitere Möglichkeit, ein geeignete olefinhaltige Einsatzgemische herzustellen, besteht darin, Raffinat I, Raffinat II oder ein ähnlich zusammengesetztes Kohlenwasserstoffgemisch in einer Reaktivkolonne zu hydroisomerisieren. Dabei kann u. a. ein Gemisch gewonnen werden, das aus 2-Butenen, geringen Anteilen 1-Buten und gegebenenfalls n-Butan sowie Isobutan und Isobuten besteht.

Die Oligomerisierung erfolgt in der Regel bei einer Temperatur im Bereich von 50 bis 200 °C, vorzugsweise 60 bis 180 °C, bevorzugt im Bereich von 60 bis 130°C, und bei einem Druck von 10 bis 70 bar, bevorzugt von 20 bis 55

bar. Sofern die Oligomerisierung in flüssiger Phase erfolgen soll, müssen die Parameter Druck und Temperatur hierfür so gewählt werden, dass der Eduktstrom (die eingesetzten Olefine oder Olefingemische) in flüssiger Phase vorliegt. Die gewichtbasierten Raumgeschwindigkeiten (Reaktandmasse pro Katalysatormasse pro Zeit; weight hourly space velocity (WHSV)) des olefinhaltigen Einsatzgemisches befinden sich im Bereich zwischen 1 g Reaktand pro g Katalysator und pro h (= 1 $h^{-1}$) und 190 $h^{-1}$, vorzugsweise zwischen 2 $h^{-1}$ und 35 $h^{-1}$, besonders bevorzugt zwischen 3 $h^{-1}$ und 25 $h^{-1}$.

In einer Ausführungsform beträgt der Grad der Dimerisierung (auch "prozentuale Selektivität bezogen auf die Dimerisierung" genannt) nach der Oligomerisierung bezogen auf das umgesetzte Edukt mindestens 60 %, weiter bevorzugt mindestens 75 %, besonders bevorzugt mindestens 80%.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach folgender allgemeiner Formel:

$$\frac{(\text{einfach verzweigte Dimere (Gew.-\%)} + 2 \text{ x zweifach verzweigte Dimere (Gew.-\%)})}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,15.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein $C_9$-Alkoholgemisch. Das $C_9$-Säuregemisch kann zur Herstellung von Schmiermitteln oder Sikkative verwendet werden. Das $C_9$-Alkoholgemisch ist Vorstufe für die Herstellung von Weichmachern, insbesondere von Di-Nonyl-phthalaten oder DINCH.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

Beispiel:

Katalysatorsynthese (erfindungsgemäßer Katalysator 1):

In den Mischbehälter eines Intensivmischers werden ein Binder (kolloidale Lösung mit Siliciumdioxid, $SiO_2$-Gehalt von ca. 30 Gew.-%), eine Nickelquelle (NiHAC-Lösung, Nickelgehalt zwischen 11 bis 12,5 Gew.-%), und amorphes Silica-Alumina (77,2 Gew.-% $SiO_2$, 12,2 Gew.-% $Al_2O_3$, Rest: Wasser, Ammoniak, Spuren weiterer Oxide (Glühverlust), mittlere Partikelgröße von 22 $\mu$m, spezifische Oberfläche von 320 $m^2$/g, Verhältnis von tetraedrisch koordinierten Gerüst-Aluminiumatomen zu oktaedrischen koordinierten Gerüst-Aluminiumatomen von 65 : 35 (kalziniert ohne Ni)) gegeben.

Nachdem alle Komponenten zugegeben wurden, wird das Gemisch für eine effektive Verteilung bei relativ geringer Drehzahl gerührt. Durch eine darauffolgende Erhöhung der Drehzahl des Rührers setzt langsam eine Verdichtung und Granulierung der Masse ein. Die Rührung wird gestoppt, sobald Granulate mit einem geeigneten Partikeldurchmesser (0,1 mm bis 7 mm) erhalten werden. Das so erhaltene Granulat wird bei etwa 120 °C getrocknet und danach mithilfe von zwei Sieben gesiebt, um zu kleine oder zu große Partikel aus dem Granulat zu entfernen.

Anschließend wird das Granulat in einem Ofen kalziniert. Für die Kalzination wird das Granulat auf eine Temperatur zwischen 500 bis 600 °C geheizt und diese Temperatur für etwa 10 bis 12 Stunden gehalten. Der mit Granulat gefüllte Ofen wird mit Stickstoff durchströmt, wobei ein Verhältnis von Volumen an Granulat zu Volumen an Stickstoff pro Stunde (Normvolumen) von mindestens 1:1000 eingehalten wird. Während der Abkühlung des Granulats auf Raumtemperatur werden ca. 6000 Vol.-ppm Luft in den Stickstoffstrom dosiert. Das abgekühlte Granulat entspricht dem fertigen Oligomerisierungskatalysator. Der so hergestellte Katalysator weist ein Verhältnis von tetraedrischen koordinierten Gerüst-Aluminiumatomen zu oktaedrischen koordinierten Gerüst-Aluminiumatomen von etwa 95 : 5 auf.

Katalysatorsynthese (nicht erfindungsgemäßer Katalysator 2):

In den Mischbehälter eines Intensivmischers werden ein Binder (Lösung aus Boehmit und einer 1-Gew.-% Salpetersäurelösung, Aluminium-Gehalt zwischen 15 bis 17 Gew.-%), eine Nickelquelle (Nickelpaste, angefeuchtetes Nickelcarbonat, Nickelgehalt zwischen 40 bis 42 Gew.-%), und amorphes Silica-Alumina (77,2 Gew.-% $SiO_2$, 12,2 Gew.-% $Al_2O_3$, Rest: Wasser, Ammoniak, Spuren weiterer Oxide (Glühverlust), mittlere Partikelgröße von 22 $\mu$m, spezifische Oberfläche von 320 $m^2/g$, Verhältnis von tetraedrisch koordinierten Gerüst-Aluminiumatomen zu oktaedrischen koordinierten Gerüst-Aluminiumatomen von 65 : 35 (kalziniert ohne Ni)) gegeben.

Silica-Alumina, Binder und feste Nickelquelle werden in dem Intensivmischer vermischt. Während der Durchmischung werden zusätzlich als flüssige Komponenten eine NiHAC-Lösung (Nickelcarbonat gelöst in konzentrierter ammoniakalischer Lösung, Nickelgehalt zwischen 11 und 12,5%) und eine Alkalimetallverbindung (Natriumcarbonat gelöst in destilliertem Wasser) über einen Trichter langsam in den Mischbehälter gegeben.

Die restliche Herstellung, umfassend Granulation, Trocknung, Siebung und Kalzination entspricht der Herstellung für Katalysator 1. Der so hergestellte Katalysator weist ein Verhältnis von tetraedrischen koordinierten Aluminiumatomen zu oktaedrischen koordinierten Aluminiumatomen von etwa 65 : 35 auf.

Einsatz der Katalysatoren in der Oligomerisierung:

Es wurden jeweils ca. 12 g des Katalysators in ein Metallrohr mit einem Innendurchmesser vom 6 mm eingefüllt. Vor und hinter dem Katalysator wurden Glasperlen mit einem Durchmesser von 2 mm gegeben, die als Vorheiz- bzw. Abkühlphase dienen. Die Oligomerisierung wurde unter Verwendung von einem Beschickungsstrom bei 30 bar und einer Belastung von 7,5 g/h Buten pro Gram Katalysator durchgeführt, wobei die Reaktionstemperatur zwischen 80 °C und 100 °C variiert wurde. Die Produkte wurden gaschromatografisch auf den Umsatz an Butenen und die Linearität der Octene analysiert. Die Zusammensetzungen des Beschickungsstroms für die Oligomerisierung zeigt die nachfolgende Tabelle 1.

Die für den Beschickungsstrom in Abhängigkeit von der Temperatur erzielten Umsätze und Selektivitäten für Katalysator 1 (erfindungsgemäß) und Katalysator 2 (nicht erfindungsgemäß), sowie die daraus resultierenden ISO-Indices sind in den Tabellen 2 und 3 angegeben.

Tabelle 1: Zusammensetzung des Beschickungsstroms

| Beschickungsstrom | |
|---|---|
| iso-Butan | 8,0 % |
| n-Butan | 15,3 % |
| trans-2-Buten | 27,9 % |
| 1-Buten | 32,7 % |
| iso-Buten | 0,9 % |
| cis-2-Buten | 15,2 % |

Tabelle 2: Umsätze und ISO-Indices bei der Oligomerisierung unter Verwendung von Katalysator 1

| Belastung (Zulauf C4-Olefine in g/h pro Masse Katalysator in g) als WSHV : 7,5 $h^{-1}$ | | | |
|---|---|---|---|
| | Temperatur | Umsatz | ISO-Index |
| | | bezogen auf C4-Olefine | |
| Katalysator 1 (erfindungsgemäß) | 80 °C | 36,2% | 1,09 |
| | 90 °C | 41,6% | 1,12 |
| | 100 °C | 44,9% | 1,10 |

Tabelle 3: Umsätze und ISO-Indices bei der Oligomerisierung unter Verwendung von Katalysator 2

| Belastung (Zulauf C4-Olefine in g/h pro Masse Katalysator in g) als WSHV : 7,5 $h^{-1}$ | | | |
|---|---|---|---|
| | Temperatur | Umsatz | ISO-Index |
| | | bezogen auf C4-Olefine | |
| Katalysator 2 (nicht erfindungsgemäß) | 80 °C | 32,6% | 1,11 |
| | 90 °C | 38,3% | 1,10 |
| | 100 °C | 40,6% | 1,08 |

Im Gegensatz zum Katalysator 2, welcher ein Verhältnis von tetraedrisch zu oktaedrisch koordiniertem Aluminium von etwa 65 : 35 aufweist, liegt das Verhältnis für den erfindungsgemäßen Katalysator bei 95 : 5. Es zeigt sich überraschenderweise, dass sich dadurch bei in etwa gleichbleibenden ISO-Indices eine erkennbare Steigerung des Umsatzes erreichen lässt.

## Patentansprüche

1. Oligomerisierungskatalysator, welcher Nickeloxid, einen Al-freien und Si-haltigen Binder (<0,1 Gew.-% Al) und ein amorphes Silica-Alumina-Supportmaterial umfasst, wobei der Katalysator eine Zusammensetzung aufweist von 15 bis 40 Gew.-% NiO, 5 bis 20 Gew.-% $Al_2O_3$, 55 bis 80 Gew.-% $SiO_2$ und 0,01 bis 1 Gew.-%, vorzugsweise 0,05 bis 0,5 Gew.-% eines Alkalimetalloxids, **dadurch gekennzeichnet, dass** der Oligomerisierungskatalysator ein Verhältnis von tetraedrisch koordinierten Gerüst-Aluminiumatomen zu oktaedrischen koordinierten Gerüst-Aluminiumatomen von 75 : 25 bis 100 : 0, bestimmt mittels $^{27}$Al-MAS-NMR, aufweist.

2. Katalysator nach Anspruch 1, wobei der Katalysator eine spezifische BET-Oberfläche von 150 bis 400 $m^2$/g, bestimmt mittels Stickstoff-Physisorption, aufweist.

3. Oligomerisierungskatalysator nach Anspruch 1 oder 2, wobei der der Oligomerisierungskatalysator Mesoporen und Makroporen aufweist.

4. Katalysator nach Anspruch 3, wobei die Mesoporen des Oligomerisierungskatalysators einen mittleren Porendurchmesser von 5 bis 15 nm, bestimmt mittels Quecksilber-Porosimetrie, aufweist.

5. Oligomerisierungskatalysator nach Anspruch 3, wobei die Makroporen des Oligomerisierungskatalysators einen mittleren Porendurchmesser von 1 bis 100 μm, bestimmt mittels Quecksilber-Porosimetrie, aufweisen.

6. Oligomerisierungskatalysator nach einem der Ansprüche 1 bis 5, wobei der Oligomerisierungskatalysator als Granulat vorliegt.

7. Oligomerisierungskatalysator nach einem der Ansprüche 1 bis 6, wobei der Oligomerisierungskatalysator einen mittleren Partikeldurchmesser (d50) von 0,1 mm bis 7 mm, bestimmt mittels bildgebender Verfahren gemäß ISO 13322-1 (Stand: 2004-12-01) und ISO 13322-2 (Stand: 2006-11-01).

8. Verfahren zur Oligomerisierung von C3- bis C6-Olefinen, wobei ein olefinhaltiges Einsatzgemisch, welches die C3- bis C6-Olefine enthält, in einer Reaktionszone über einen Katalysator geleitet wird, wobei der Katalysator nach einem der Ansprüche 1 bis 7 zur Katalyse der Oligomerisierungsreaktion eingesetzt wird.

9. Verfahren zur Oligomerisierung nach Anspruch 8, wobei C3- bis $C_5$-Olefine oligomerisiert werden und das olefinhaltige Einsatzgemisch die C3- bis $C_5$-Olefine enthält.

10. Verfahren zur Oligomerisierung nach Anspruch 8, wobei C4-Olefine oligomerisiert werden und das olefinhaltige Einsatzgemisch die C4-Olefine enthält.

11. Verfahren zur Oligomerisierung nach einem der Ansprüche 8 bis 10, wobei das olefinhaltige Einsatzgemisch weniger als 2 Gew.-% verzweigte Olefine enthält.

**12.** Verfahren nach einem der Ansprüche 8 bis 11, wobei die Oligomerisierung in flüssiger Phase stattfindet.

**13.** Verfahren zur Oligomerisierung nach einem der Ansprüche 8 bis 12, wobei die Oligomerisierung bei einem Druck von 10 bis 70 bar und einer Temperatur von 50 bis 200 °C durchgeführt wird, unter der Voraussetzung, dass, sofern die Oligomerisierung in flüssiger Phase durchgeführt wird, die Parameter Druck und Temperatur so gewählt werden, dass der Eduktstrom in flüssiger Phase vorliegt.

**14.** Verfahren zur Oligomerisierung nach einem der Ansprüche 8 bis 13, wobei die gewichtsbasierte Raumgeschwindigkeit (WHSV) des olefinhaltigen Einsatzgemisches im Bereich zwischen 1 $h^{-1}$ und 190 $h^{-1}$ liegt.

Europäisches Patentamt
European Patent Office
Office européen des brevets

# EUROPÄISCHER RECHERCHENBERICHT

Nummer der Anmeldung
EP 19 16 2229

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2009 027408 A1 (EVONIK OXENO GMBH [DE]) 5. Januar 2011 (2011-01-05)<br>* Absätze [0003], [0037]; Ansprüche 7,8; Beispiele 1,2 *<br>----- | 1-14 | INV.<br>B01J21/12<br>B01J23/04<br>B01J23/755<br>B01J35/00<br>B01J37/00<br>B01J37/02<br>C07C2/10 |
| X | WO 2014/123243 A1 (JX NIPPON OIL & ENERGY CORP [JP]) 14. August 2014 (2014-08-14)<br>* Seite 5; Ansprüche 1-5; Beispiele 1-3; Tabelle 2 *<br>----- | 1-14 | |
| X | US 3 557 242 A (SAMPSON ROY JOHN ET AL) 19. Januar 1971 (1971-01-19)<br>* Ansprüche 1,3; Beispiel 6(B)(ii) *<br>----- | 1-14 | |
| A | FR 3 054 455 A1 (IFP ENERGIES NOW [FR]) 2. Februar 2018 (2018-02-02)<br>* Seite 3, Zeilen 26-29; Beispiele 1B,2B,4,5 *<br>* Seite 11, Zeilen 9-24 *<br>----- | 1-14 | RECHERCHIERTE SACHGEBIETE (IPC) |
| A | WO 95/14647 A1 (BASF AG [DE]; VICARI MAXIMILIAN [DE]; POLANEK PETER [DE]) 1. Juni 1995 (1995-06-01)<br>* Seite 5, Zeilen 23-28 *<br>* Seite 6, Zeilen 28-33 *<br>* Beispiele *<br>-----<br>-/-- | 1-14 | B01J<br>C07C |

Der vorliegende Recherchenbericht wurde für alle Patentansprüche erstellt

1

| Recherchenort | Abschlußdatum der Recherche | Prüfer |
|---|---|---|
| München | 19. August 2019 | Goebel, Matthias |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 19 16 2229

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | MOUSSA SARA ET AL: "Heterogeneous oligomerization of ethylene to liquids on bifunctional Ni-based catalysts: The influence of support properties on nickel speciation and catalytic performance", CATALYSIS TODAY, ELSEVIER, AMSTERDAM, NL, Bd. 277, 11. Januar 2016 (2016-01-11), Seiten 78-88, XP029743467, ISSN: 0920-5861, DOI: 10.1016/J.CATTOD.2015.11.032<br>* Zusammenfassung *<br>* Seite 80 - Seite 82; Abbildungen 1,2; Tabellen 1,2 *<br>* Seite 86, linke Spalte; Tabelle 4 *<br>----- | 1-14 | |
| A | US 7 572 946 B2 (INST FRANCAIS DU PETROLE [FR]) 11. August 2009 (2009-08-11)<br>* Anspruch 1; Beispiel 2 *<br>----- | 1-14 | |
| A | US 6 733 657 B2 (INST FRANCAIS DU PETROLE [FR]) 11. Mai 2004 (2004-05-11)<br>* Ansprüche 1,2; Beispiele 1-5 *<br>----- | 1-14 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 19. August 2019 | Goebel, Matthias |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........
& : Mitglied der gleichen Patentfamilie, übereinstimmendes Dokument

EPO FORM 1503 03.82 (P04C03)

Seite 2 von 2

## ANHANG ZUM EUROPÄISCHEN RECHERCHENBERICHT ÜBER DIE EUROPÄISCHE PATENTANMELDUNG NR.

EP 19 16 2229

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-08-2019

| Im Recherchenbericht angeführtes Patentdokument | Datum der Veröffentlichung | Mitglied(er) der Patentfamilie | Datum der Veröffentlichung |
|---|---|---|---|
| DE 102009027408 A1 | 05-01-2011 | DE 102009027408 A1 | 05-01-2011 |
| | | WO 2011000697 A1 | 06-01-2011 |
| WO 2014123243 A1 | 14-08-2014 | JP 2014151253 A | 25-08-2014 |
| | | WO 2014123243 A1 | 14-08-2014 |
| US 3557242 A | 19-01-1971 | BE 729350 A | 04-09-1969 |
| | | DE 1911030 A1 | 25-09-1969 |
| | | FR 2003215 A1 | 07-11-1969 |
| | | GB 1195307 A | 17-06-1970 |
| | | NL 6903344 A | 08-09-1969 |
| | | US 3557242 A | 19-01-1971 |
| FR 3054455 A1 | 02-02-2018 | KEINE | |
| WO 9514647 A1 | 01-06-1995 | DE 4339713 A1 | 24-05-1995 |
| | | EP 0730567 A1 | 11-09-1996 |
| | | ES 2122510 T3 | 16-12-1998 |
| | | JP 3544980 B2 | 21-07-2004 |
| | | JP H09505618 A | 03-06-1997 |
| | | US 5849972 A | 15-12-1998 |
| | | WO 9514647 A1 | 01-06-1995 |
| US 7572946 B2 | 11-08-2009 | DK 1616846 T3 | 25-01-2016 |
| | | EP 1616846 A1 | 18-01-2006 |
| | | FR 2873116 A1 | 20-01-2006 |
| | | JP 4958413 B2 | 20-06-2012 |
| | | JP 2006028519 A | 02-02-2006 |
| | | US 2006063955 A1 | 23-03-2006 |
| US 6733657 B2 | 11-05-2004 | AT 510617 T | 15-06-2011 |
| | | DK 1353747 T3 | 19-09-2011 |
| | | EP 1353747 A1 | 22-10-2003 |
| | | FR 2819430 A1 | 19-07-2002 |
| | | JP 2004535268 A | 25-11-2004 |
| | | KR 20030071818 A | 06-09-2003 |
| | | US 2002160911 A1 | 31-10-2002 |
| | | WO 02055192 A1 | 18-07-2002 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9514647 A1
- US 7572946 B2
- US 6733657 B2